# EUROPÄISCHE PATENTANMELDUNG

(11) **EP 1 829 878 A1**
(43) Veröffentlichungstag der Anmeldung: **05.09.2007**
(21) Anmeldenummer: 06004218.1
(22) Anmeldetag: 02.03.2006
(51) Int. Cl.: C07D 489/02, A61K 31/485, A61P 25/04

(54) **Neue Morphinverbindungen für pharmazeutische Zusammensetzungen**

(71) Anmelder: Universitätsklinikum Freiburg, 79106 Freiburg (DE)
(72) Erfinder: Trittler, Rainer, Dr., 79114 Freiburg (DE)
(74) Vertreter: Keller, Günter

(57) **Zusammenfassung**

Die Erfindung betrifft neue Morphinverbindungen der Formel: 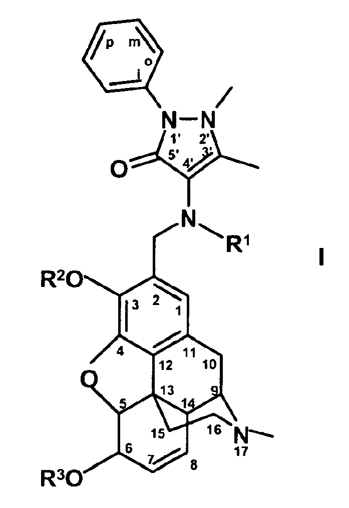 wobei R¹ einen C₁₋₆-Alkylrest darstellt und die Reste R² und R³ unabhängig voneinander aus der Gruppe ausgewählt sind, die aus Wasserstoffatomen, Methyl- und Acetylgruppen besteht.

## Beschreibung

Die Erfindung betrifft neue Morphinverbindungen der Formel I, die aus Reaktionsgemischen hergestellt werden können, die Morphin-Derivate und Pyrazol-Derivate, wie beispielsweise Metamizol, enthalten. Des Weiteren betrifft die Erfindung pharmazeutische Zusammensetzungen, die Verbindungen der Formel I enthalten.

Metamizol als Pyrazolderivat gehört der Gruppe der Phenazone an und stellt ein antipyretisch wirkendes Analgetikum dar. Die analgetische Wirkung erfolgt durch eine Dämpfung der zentralen Schmerzperzeption in Folge einer Aktivierung von Neuronen im schmerzhemmenden System. Ebenso werden Morphin-Derivate als Opioidanalgetika in der Schmerztherapie eingesetzt.

Die Stabilität von Metamizol, N-Methyl-N-(2,3-dimethyl-5-oxo-1-phenyl-3-pyrazolin-4-yl) aminomethansulfonsäure, bzw. dessen pharmazeutisch unbedenklichen Salzen ist pHabhängig. Im neutralen und basischen Milieu sind Metamizol bzw. dessen pharmazeutisch unbedenklichen Salze stabil. Im sauren Milieu allerdings erfolgt sehr rasch eine Hydrolyse, die sich durch eine Gelbfärbung zeigt.

EP 1 150 660 offenbart die Herstellung von Brausetabletten mit Metamizol, wobei trotz des sauren pH-Wertes der Brauseformulierung eine akzeptable Stabilität des Wirkstoffs erreicht wird.

Ein Problem von Morphin-Derivaten in der Schmerztherapie ist die potentielle Suchtgefahr von einigen Verbindungen. Gerade das schnelle Passieren der Blut-Hirn-Schranke kann zur Abhängigkeit des Patienten von diesen Stoffen führen. Im Hinblick auf die Nebenwirkung von Morphin beschreibt die US 5.521.178 die Verwendung von Flupirtin als schmerzlinderndes Mittel ohne Suchtpotential. Die Verwendung von Pro-Drugs, die ein Morphin-Derivat langsam entstehen lassen, könnte ebenfalls dazu beitragen, das Suchtpotential zu verringern.

Morphinalkaloide können auch als quaternäre Stickstoffverbindungen eingesetzt werden. Die Herstellung dieser Verbindungen ist beispielsweise in WO 2004/043964 beschrieben.

Die Herstellung von Arzneimitteln mit dem Wirkstoff Metamizol in Kombination mit anderen Wirkstoffen bzw. Inhaltsstoffen ist aufgrund des Problems der Stabilität von Metamizol eingeschränkt. Eine Arzneimittelzusammensetzung die beispielsweise Metamizol und Morphin enthält, gibt es bislang nicht.

Durch Versuche konnte gezeigt werden, dass Morphin-Derivate mit Pyrazol-Derivaten, insbesondere Metamizol, unter geeigneten Bedingungen, wie z.B. geeignetem pH-Wert und Reaktionstemperatur, Reaktionen eingehen und dabei neue Morphinverbindungen entstehen. Die Produkte dieser Reaktionen sind Gegenstand der vorliegenden Erfindung.

Bei entsprechenden Bedingungen findet keine Umsetzung statt. So konnten in einem Gemisch aus Morphin und Metamizol bei einer Lagerung von 2 Wochen bei Raumtemperatur keine neuen Morphinverbindungen nachgewiesen werden.

Es ist eine Aufgabe der vorliegenden Erfindung, diese neuen Verbindungen für pharmazeutische Zusammensetzungen bereitzustellen. Verbindungen der Formel 1 können beispielsweise als Pro-Drug z.B. in der Schmerztherapie verwendet werden. Ein Vorteil kann darin liegen, dass eine pharmazeutische Zusammensetzung bereitgestellt werden kann, die Morphin-Derivate zusammen mit Pyrazol-Derivaten als aktive Wirkstoffe in Form einer Pro-Drug enthält, wobei diese pharmazeutische Zusammensetzung auch bei längerer Lagerung stabil bleibt und ein konstantes Mengenverhältnis der Wirkstoffe enthält.

Ein weiterer Vorteil der neuen Morphinverbindungen kann auch in einer Retardwirkung liegen, sei es aufgrund pharmakokinetischer oder pharmakodynamischer Effekte.

Auch eine Verringerung der Nebenwirkungen gegenüber den Wirkstoffen Morphin und Metamizol könnte eine vorteilhafte Eigenschaft der neuen Verbindungen sein, da z.B. die Verteilung der Wirkstoffe auf die Kompartimente des menschlichen Körpers unterschiedlich sein wird.

Des Weiteren könnten die erfindungsgemäßen Verbindungen eine neue pharmakodynamische Wirkung am Morphinrezeptor aufweisen.

Eine pharmazeutische Zusammensetzung kann ebenfalls Salze, beispielsweise in Form von quaternären N-Derivaten, von Verbindungen der Formel I enthalten.

Die vorliegende Erfindung betrifft somit Verbindungen der Formel I 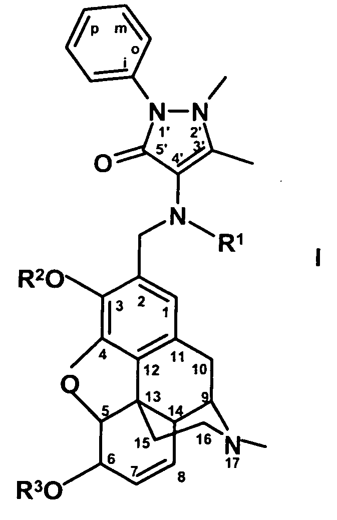 wobei R¹ einen C₁₋₆-Alkylrest, wie beispielsweise den Methyl-, Ethyl-, Propyl-, oder Buthylrest, darstellt und die Reste R² und R³ unabhängig voneinander aus der Gruppe ausgewählt sind, die aus Wasserstoffatomen, Methyl- und Acetylgruppen besteht.

Bevorzugt sind Verbindungen der Formel I, wobei R¹ eine Methylgruppe darstellt und die Reste R² und R³ unabhängig voneinander aus der Gruppe ausgewählt sind, die aus Wasserstoffatomen, Methyl- und Acetylgruppen besteht.

Besonders bevorzugt sind Verbindungen der Formel I, wobei R¹ eine Methylgruppe darstellt und die Reste R² und R³ die gleichen Reste darstellen und aus der Gruppe ausgewählt sind, die aus Wasserstoffatomen, Methyl- und Acetylgruppen besteht.

Des Weiteren betrifft die Erfindung Verbindungen der Formel I, wobei R¹ eine Methylgruppe darstellt und die Reste R² und R³ Wasserstoffatome darstellen.

Die vorliegende Erfindung betrifft auch eine pharmazeutische Zusammensetzung, die eine oder mehrere Verbindungen der Formel I oder Salze dieser Verbindungen umfasst, wobei zusätzlich andere Zusatzstoffe und/oder Wirkstoffe enthalten sein können. Als Salze kommen übliche, pharmazeutisch annehmbare Verbindungen in Betracht.

Bevorzugt ist eine pharmazeutische Zusammensetzung, die eine Verbindung der Formel I oder ein Salz dieser Verbindung umfasst, wobei R¹ eine Methylgruppe darstellt und die Reste R² und R³ Wasserstoffatome darstellen.

Weiter bevorzugt sind pharmazeutische Zusammensetzungen, wobei die Verbindung der Formel I in einer Stoffmenge von mindestens 20 Gew.-%, bevorzugt mindestens 50 Gew.-% und ganz besonders bevorzugt mindestens 90 Gew.-% bezogen auf die Gesamtzusammensetzung enthalten ist.

Die Erfindung betrifft des Weiteren die Verwendung einer Verbindung der Formel I zur Herstellung einer Infusionslösung, wobei die Reste R¹, R² und R³ wie im Vorangegangenen definiert sind.

Besonders bevorzugt ist die Verwendung einer Verbindung der Formel I zur Herstellung einer pharmazeutischen Zusammensetzung zur Behandlung von Schmerzen, wobei die Reste R¹, R² und R³ wie oben definiert sind.

Die Erfindung betrifft zudem ein Verfahren zur Herstellung von Verbindungen der Formel I: 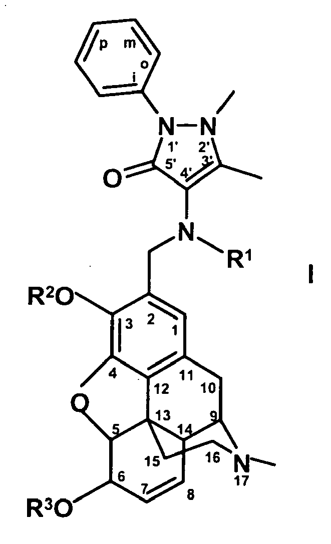 worin eine Verbindung der Formel II, oder ein Salz davon 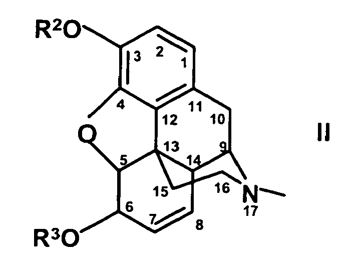 und eine Verbindung der Formel III, oder ein Salz davon 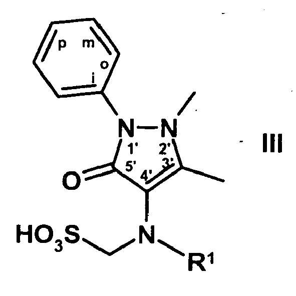 umgesetzt werden, und wobei R¹ einen C₁₋₆-Alkylrest darstellt und die Reste R² und R³ unabhängig voneinander aus der Gruppe ausgewählt sind, die aus Wasserstoffatomen, Methyl- und Acetylgruppen besteht.

Bevorzugt ist das Verfahren, wobei R¹ eine Methylgruppe darstellt und die Reste R² und R³ Wasserstoffatome darstellen.

Für die Synthese der Verbindungen der Formel I werden bevorzugt Verbindungen der Formel II und III als Ausgangsstoffe verwendet. Verbindungen der Formel II werden als Morphin-Derivate bezeichnet. Die Verbindung der Formel II wird als Morphin bezeichnet, falls die beiden Reste R² und R³ Wasserstoffatome sind, bzw. als Codein falls der Rest R² ein Wasserstoffatom und R³ eine Methylgruppe ist, bzw. als Heroin, falls R² und R³ Acetylgruppen sind. Eine bevorzugte Verbindung der Formel II ist das Morphin.

Verbindungen der Formel III sind Pyrazol-Derivate bzw. Derivate der N-Methyl-N-(2-methyl-5-oxo)-1-phenyl-3-pyrazolin-4-yl)aminomethansulfonsäure, wobei sich in 3-Position des Pyrazolinringes (Rest R¹) ein C₁₋₆-Alkylrest befindet, der 1 bis 6 Kohlenstoffatome enthält. Besonders bevorzugt ist eine Methylgruppe in 3-Position (R¹ = Methyl), die entsprechende Verbindung wird als Metamizol bezeichnet.

Die Herstellung der Verbindungen der Formel I erfolgt durch Mischen der Verbindungen der Formel II und III oder deren Salze und anschließender Lagerung bei einer Temperatur, die höher als 37°C ist (ohne Lichtschutz, bei einer Reaktionszeit von ca. 6 Wochen), bevorzugt liegt sie bei 45°C, weiter bevorzugt bei 65°C und am meisten bevorzugt bei 80°C.

Hierbei stellt R¹ einen C₁₋₆-Alkylrest dar und die Reste R² und R³ sind unabhängig voneinander aus der Gruppe ausgewählt, die aus Wasserstoffatomen, Methyl- und Acetylgruppen besteht. Ein für die Reaktion geeignetes Lösungsmittel kann beispielsweise eine wässrige Lösung sein. Der pH-Wert des Reaktionsgemisches muss zu Reaktionsbeginn kleiner als 6,0 sein, bevorzugt ist der pH-Wert kleiner oder gleich 5,7 und am meisten bevorzugt ist er kleiner als 5,0. Das Einstellen des pH-Wertes kann nach den Methoden erfolgen, die dem Fachmann dafür bekannt sind. Das Reaktionsende kann beispielsweise durch Flüssigkeitschromatographie gekoppelt mit Massenspektroskopie (LC-MS, Liquid Chromatography with Mass Spectroscopy) bestimmt werden und ist dann erreicht, wenn der gewünschte Umsatz festgestellt wird. Die Aufarbeitung des Reaktionsgemisches kann durch übliche Verfahren erfolgen. Die anschließende Reinigung des Reaktionsgemisches und Isolierung der neuen Morphinverbindungen kann durch Hochleistungs-Flüssigkeitschromatographie (HPLC)-Methoden, oder auch durch Säulenchromatographie erfolgen. Die Identifizierung und Isolierung der Produkte kann zudem durch Massenspektroskopie (LC-MS) sowie NMR-Spektroskopie erfolgen.

In einem weiteren Herstellungsverfahren der Verbindungen der Formel I wird die Synthese in Wasser oder einem organischen Lösungsmittel durchgeführt. Hierbei stellt R¹ einen C₁₋₆-Alkylrest dar und die Reste R² und R³ sind unabhängig voneinander aus der Gruppe ausgewählt, die aus Wasserstoffatomen, Methyl- und Acetylgruppen besteht. Der pH-Wert des Reaktionsgemisches muss zu Reaktionsbeginn kleiner als 6,0 sein, bevorzugt ist der pH-Wert kleiner oder gleich 5,7 und am meisten bevorzugt ist er kleiner als 5,0. Das Einstellen des pH-Wertes kann nach den Methoden erfolgen, die dem Fachmann dafür bekannt sind. Die Reaktionstemperatur ist größer als 37°C, bevorzugt liegt sie bei 45°C, weiter bevorzugt bei 65°C und am meisten bevorzugt bei 80°C. Das Reaktionsende kann beispielsweise durch Massenspektroskopie (LC-MS) bestimmt werden und ist dann erreicht, wenn der gewünschte Umsatz festgestellt wird. Die Aufarbeitung des Reaktionsgemisches kann durch übliche Verfahren erfolgen. Die anschließende Auftrennung des Reaktionsgemisches und Isolierung der neuen Morphinverbindungen kann durch HPLC-Methoden, oder auch durch Säulenchromatographie erfolgen. Die Identifizierung und Isolierung der Produkte kann zudem durch LC-MS und NMR(Nuclear Magnetic Resonance)-Spektroskopie erfolgen.

Ein drittes Herstellungsverfahren verwendet als Ausgangsverbindungen Morphin-Derivate der Formel II, Formaldehyd und Verbindungen der Formel IV. Die Reaktionsbedingungen, die Aufarbeitung der Reaktionsgemische und die Reinigung der Produkte können den beiden anderen Verfahren entnommen werden.

Die Entstehung der Verbindungen der Formel I lässt sich chemisch wie folgt erklären: Unter sauren Bedingungen können Verbindungen der Formel III, unter Entstehung von Formaldehyd, zersetzt werden. Beispielsweise zersetzt sich Metamizol (R¹ ist eine Methylgruppe) zu 4-Methylaminophenazon (Formel IV).

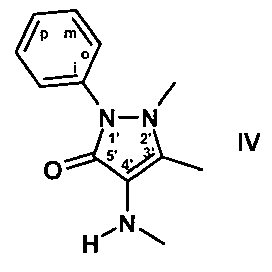

In Anschluss daran erfolgt eine Mannich-Reaktion der Morphin-Derivate (Formel II) als CHacider Komponente mit Formaldehyd und den Verbindungen der Formel IV. Die Mannich-Reaktion von Morphin als CH-acide Komponente mit Formaldehyd und anderen Aminen (z.B. Diethylamin) wurde bereits von Görlitzer und Weltrowski beschrieben (Beiträge zur Chemie und Analytik von Morphin, Sonderdruck aus Abhandlungen der Braunschweigischen Wissenschaftlichen Gesellschaft, J. Cramer Verlag Braunschweig 2002). Weitere Unterstützung findet dieser Reaktionsmechanismus durch die von Eger, Troschütz und Roth entdeckte Arzneistoffinkompatibilität von Methenamin und Phenanzon, hierbei läuft ebenfalls eine Mannich-Reaktion unter milden Bedingungen ab (Eger K, Troschütz R, Roth H: Arzneistoffanalyse, 4. Auflage, Seite 293, Deutscher Apothekerverlag Stuttgart 1999).

Bei Applikation einer frisch zubereiteten Mischung von Morphin-Derivaten mit beispielsweise Metamizol, ist im menschlichen Körper keine Reaktion zu Verbindungen der Formel I in relevanten Mengen zu erwarten. Zum einen wird der dazu notwendige Formaldehyd vom menschlichen Körper sehr schnell metabolisiert und steht nicht mehr für die Mannich-Reaktion zu Verfügung. Andererseits ist die Verweildauer der Stoffe im Körper zu kurz, im Vergleich zu einer Reaktionszeit von mehreren Wochen, sodass keine relevante Menge der Verbindung der Formel I entstehen kann.

In einer besonders bevorzugten Ausführungsform der Erfindung werden Metamizol (Formel IV) und Morphin (Formel V) als Ausgangsverbindungen verwendet.

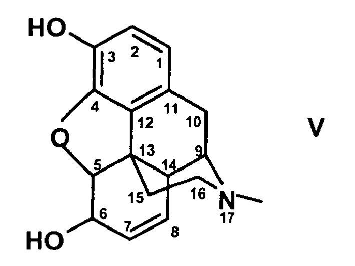

Bei der Reaktion eines Gemisches von Metamizol mit Morphin entsteht bei leicht saurem pH-Wert die Verbindung der Formel VI (R¹ ist eine Methylgruppe, die Reste R² und R³ sind Wasserstoffatome), welche im Nachfolgenden als "Metamorphin" bezeichnet wird.

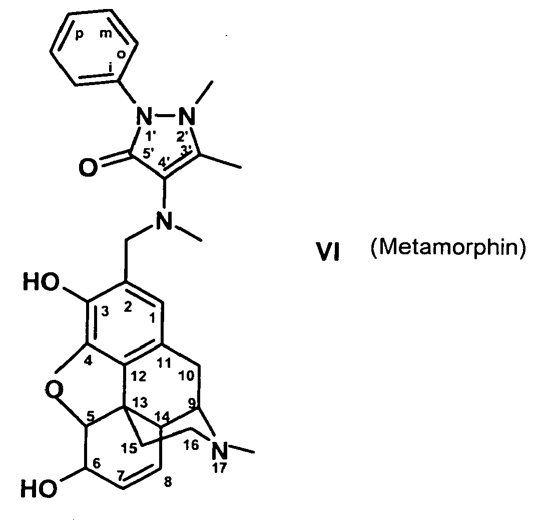

Eine weitere Ausführungsform der Erfindung betrifft eine pharmazeutische Zusammensetzung, die eine oder mehrere Verbindungen der Formel I enthält, wobei R¹ einen C₁₋₆-Alkylrest darstellt und die Reste R² und R³ unabhängig voneinander aus der Gruppe ausgewählt sind, die aus Wasserstoffatomen, Methyl- und Acetylgruppen besteht.

Die Herstellung der Verbindung der Formel I erfolgt nach einem der oben beschriebenen Verfahren, ist jedoch nicht auf diese Verfahren eingeschränkt. Die pharmazeutische Zusammensetzung kann auch eine Mischung einer Verbindung der Formel I oder dessen Salz mit Verbindungen der Formel II und III (oder deren Salze) bzw. deren Abbau-/ Hydrolyseprodukten, die zur Synthese der Verbindung der Formel I verwendet werden können, sein. Bevorzugt enthalten diese pharmazeutischen Zusammensetzungen die Verbindungen der Formel I in einer Stoffmenge von mind. 20 % der Wirkstoffe, besonders bevorzugt enthalten sie mind. 40 %, weiter bevorzugt enthalten sie mind. 50%, am meisten bevorzugt mind. 80 Gew.-% bezogen auf das Gesamtgewicht der Zusammensetzung. Die erfindungsgemäße pharmazeutische Zusammensetzung kann weitere Wirkstoffe oder bevorzugt nur Wirkstoffe der Formel I enthalten. Die pharmazeutische Zusammensetzung kann in Form einer Infusion- oder Injektionslösung intravenös oder subkutan, aber auch in jeder anderen Applikationsform wie beispielsweise Tabletten, Zäpfchen oder Tropfen, oral oder rektal, verabreicht werden.

Eine andere Ausführungsform enthält die Verbindung Metamorphin mit der Formel VI (R¹ ist eine Methylgruppe, die Reste R² und R³ sind Wasserstoffatome) in der pharmazeutischen Zusammensetzung. Die Herstellung der Verbindung der Formel VI erfolgt bevorzugt nach einem der oben beschriebenen Verfahren, ist jedoch nicht auf diese Verfahren eingeschränkt. Eine derartige Zusammensetzung kann weitere Wirk- und Inhaltsstoffe enthalten. Bevorzugt enthalten diese pharmazeutischen Zusammensetzungen die Verbindung der Formel VI in einer Stoffmenge von mind. 20 % der Wirkstoffe, besonders bevorzugt enthalten sie mind. 40 %, weiter bevorzugt enthalten sie mind. 50%, am meisten bevorzugt mind. 80% bezogen auf das Gesamtgewicht der Zusammensetzung. Die pharmazeutische Zusammensetzung kann in Form einer Infusion- oder Injektionslösung intravenös oder subkutan, aber auch in jeder anderen Applikationsform wie beispielsweise Tabletten, Zäpfchen oder Tropfen, oral oder rektal, verabreicht werden.

Die nachfolgenden Beispiele dienen der Erläuterung der Erfindung.

### Beispiel 1:

### a) Synthese

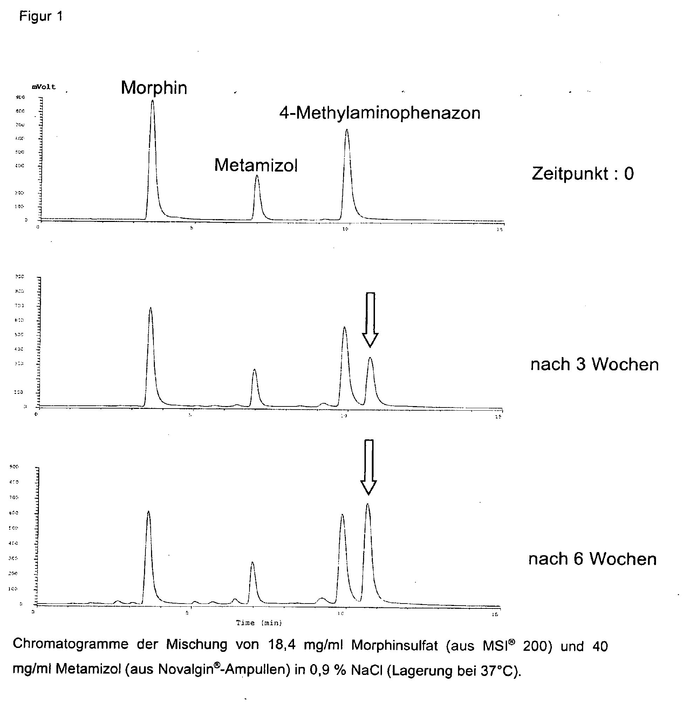

Ein Infusionsbeutel wurde mit einer Mischung von 18,4 mg/ml Morphinsulfat (aus MSI^{®} 200) und 40 mg/ml Metamizol (aus Novalgin^{®}-Ampullen) in 0,9 % NaCl befüllt und anschließend bei 37°C im Brutschrank (ohne Lichtschutz) gelagert. Nach 6 Wochen bildete sich die neue Substanz "Metamorphin" mit einer Ausbeute von ca. 50% (bezogen auf den Ausgangsgehalt des Morphins). Siehe diesbezüglich auch die in Figur 1 abgebildeten Chromatogramme.

### b) Analyse mit HPLC

Aus dem Infusionsbeutel wurden Proben unmittelbar nach Herstellung sowie nach 3 und 6 Wochen entnommen und jeweils unmittelbar danach bei -30°C eingefroren. Zur Analyse wurden die Proben aufgetaut und danach sofort mittels HPLC mit unten beschriebener Methode aufgetrennt. Mit dieser Methode werden alle Ausgangsstoffe sowie auch die neue Substanz "Metamorphin" vollständig getrennt. Figur 1 zeigt Chromatogramme der bei 37°C gelagerten Mischung von 18,4 mg/ml Morphinsulfat (aus MSI^{®} 200) und 40 mg/ml Metamizol (aus Novalgin^{®}-Ampullen) in 0,9% NaCl in Abhängigkeit von der Reaktionsdauer.

### Geräte:

HPLC-Pumpe K-1001 (Knauer), Autosampler SL 10AD VP (Shimadzu), Säulenofen CTO-10AS VP (Shimadzu), Diodenarraydetektor K-2700 (Knauer)

### Säule:

### Merck Purospher STAR, RP 18e,125x4mm

### Gradient:

| | | |
|---|---|---|
| 0min | 95% Phosphatpuffer pH5 | 5% Acetonitril |
| 10min | 70% Phosphatpuffer pH5 | 30% Acetonitril |
| 10,01min | 95% Phosphatpuffer pH5 | 5% Acetonitril |

### Fluss: 1ml/min

### c) Analyse mit LC-MS

Die HPLC-Methode wurde auf das LC-MS (esquire 3000, Bruker Daltronics) übertragen und die Masse der neuen Verbindung ermittelt.

### Säule:

### Merck Purospher STAR, RP 18e,125x4mm

### Gradient:

| | | |
|---|---|---|
| 0min | 95% Ammoniumformiatpuffer pH5 | 5% Acetonitril |
| 20min | 70% Ammoniumformiatpuffer pH5 | 30% Acetonitril |
| 20,01 min | 95% Ammoniumformiatpuffer pH5 | 5% Acetonitril |

Fluss: 0,5ml/min, Ende nach 30min

### MS-Bedingungen:

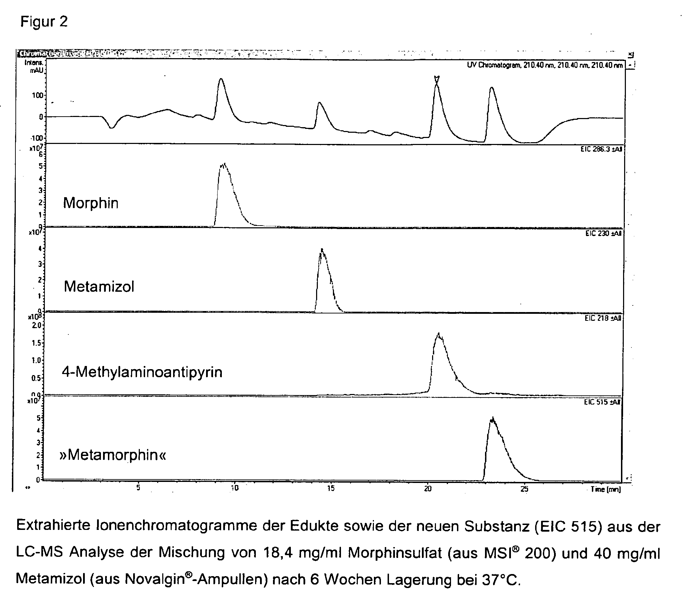

Es wurde mit ESI (Elektronen-Stoß-lonisation) im Positivmodus gearbeitet (Trap Drive 36.2, Octopol RF Amplitude 149.8 Vpp, Lens 2 -60 Volt, Capillary Exit 112.4 Volt, Dry Temp. 250°C, Nebulizer 30.0 psi, Dry Gas 12.00l/min, HV Capillary 4000 V, HV End Plate Offset - 500 V). Die Einstellungen in der Ionenfalle waren: Rolling on, Rolling Averages 2cts, Scan Begin 50 m/z, Scan End 1000 m/z, Averages 5 Spectra, Max. Accu Time 200000 µs, ICC Target 40000, Charge Control on. Figur 2 zeigt die extrahierten lonenchromatogramme der Edukte sowie der neuen Substanz. Zur besseren Vergleichbarkeit ist auch das UV-Chromatogramm dargestellt.

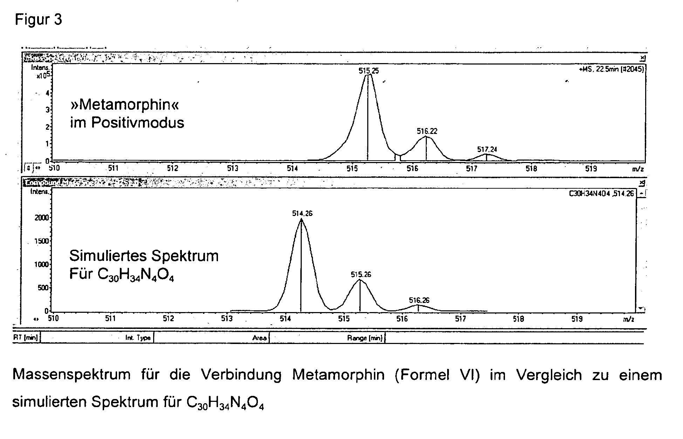

Da im Positivmodus gemessen wurde, ist die Masse der neuen Substanz um ein Proton leichter, d.h. 514 m/z. Das Massenspektrum der Substanz stimmt gut mit einem simulierten Massenspektrum einer Verbindung mit der Summenformel C₃₀H₃₄N₄O₄ überein, siehe Figur 3.

### d) Isolierung des Produkts

Die Isolierung wurde an einer Agilent LC-MS Anlage mit Massendetektor Modell SL G1946G mit Splitter G1968D und einem Fraktionssammler G1364A durchgeführt. Die Probeninjektion erfolgte mittels einem Agilent Autosampler G1313A (Injektionsvolumen 1800µl).

### Säule:

### Zorbax SB-C18, 9,4 x 150mm, 5µm

### Gradient:

| | | |
|---|---|---|
| 0min | 95% Ammoniumformiatpuffer pH5 | 5% Acetonitril |
| 15min | 70% Ammoniumformiatpuffer pH5 | 30% Acetonitril |
| 20min | 70% Ammoniumformiatpuffer pH5 | 30% Acetonitril |
| 20,01min | 5% Ammoniumformiatpuffer pH5 | 95% Acetonitril |
| 23min | 5% Ammoniumformiatpuffer pH5 | 95% Acetonitril |
| 23,01min | 95% Ammoniumformiatpuffer pH5 | 5% Acetonitril |

Fluss: 3,5ml/min, Ende nach 29min

### MS-Bedingungen:

Es wurde mit ESI im Positivmodus gearbeitet und eine massenbasierte (Masse 514, d.h. m/z = 515) Sammlung mit Hilfe des Splitters (Splittingverhältnis 2900:1) durchgeführt. (Dry Temp. 300°C, Nebulizer 35.0 psi, Dry Gas 12.001/min, HV Capillary 3000 V, Fragmentorspannung 100V).

Die gesammelte Fraktion wurde mittels eines Rotationsverdampfers vakuumgetrocknet und in Methanol aufgenommen. Die Methanolphase wurde dann in einem neuen Rundkolben nochmals eingedampft. Damit konnten ca. 300 mg der neuen Substanz isoliert werden.

### e) Charakterisierung des Produkts durch NMR-Experimente

Tabelle 1 zeigt die Ergebnisse des ¹³C-NMR sowie des ¹H-NMR, das Nummerierungschema der Verbindung ist der Formel VI zu entnehmen.

Des Weiteren wurden NOE (Nuclear-Overhauser-Enhancement)-Signale gemessen, insbesondere zwischen: H-1 und 2-CH₂, H-10 (2.81), 4'-NMe (schwach); 4'-NMe und 2-CH₂, 3'-Me; 3'-Me und 2'-Me sowie 2'-Me und o.

**Tabelle1:**

| Nummer des Kohlenstoffatoms | ¹³C-NMR (δ, ppm) ¹³C-NMR (δ, ppm) | ¹H-NMR (δ, ppm) ¹H-NMR (δ, ppm) |
|---|---|---|
| 1 | 122.3 | 6.47 (s) |
| 2 | 127.8 | |
| 3 | 140.4 | |
| 4 | 147.8 | |
| 5 | 92.4 | 4.90 (m) |
| 6 | 67.6 | 4.25 (m) |
| 7 | 135.5 | 5.70 (d, 9.8 Hz) |
| 8 | 126.7 | 5.32 (d, 9.8 Hz) |
| 9 | 61.8 | 4.05 (m) |
| 10 | 23.2 | 2.81 (m) |
| | | 3.20 (m) |
| 11 | 123.1 | |
| 12 | 130.1 | |
| 13 | 43.3 | |
| 14 | 39.1 | 3.01 (m) |
| 15 | 33.7 | 2.00 (m) |
| | | 2.35 (m) |
| 16 | 47.9 | 3.01 (m) |
| | | 3.24 (m) |
| 17-Me | 41.5* | 2.91 (s) |
| 2-CH₂ | 57.6 | 4.12 (s) |
| 4'-NMe | 41.6* | 2.75(s) |
| 4' | 121.0 | |
| 3' | 152.0 | |
| 3'-Me | 10.6 | 2.03 (s) |
| 2'-Me | 35.8 | 3.01 (s) |
| i | 135.6 | |
| o | 126.5 | 7.35 (d, 7.4 Hz) |
| m | 130.5 | 7.50 (t, 7.9 Hz) |
| p | 128.9 | 7.39 (t, 7.5 Hz) |

| | | |
|---|---|---|
| *oder umgekehrt. | | |

## Patentansprüche

1. Verbindung der Formel I: 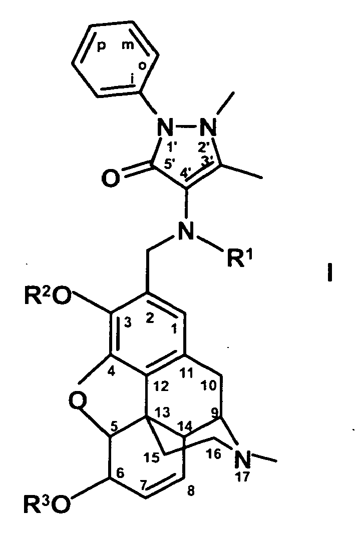 wobei R¹ einen C₁₋₆-Alkylrest darstellt und die Reste R² und R³ unabhängig voneinander aus der Gruppe ausgewählt sind, die aus Wasserstoffatomen, Methyl- und Acetylgruppen besteht.

2. Verbindung nach Anspruch 1, wobei R¹ eine Methylgruppe darstellt und die Reste R² und R³ unabhängig voneinander aus der Gruppe ausgewählt sind, die aus Wasserstoffatomen, Methyl- und Acetylgruppen besteht.

3. Verbindung nach Anspruch 1, wobei R¹ eine Methylgruppe darstellt und die Reste R² und R³ die gleichen Reste darstellen und aus der Gruppe ausgewählt sind, die aus Wasserstoffatomen, Methyl- und Acetylgruppen besteht.

4. Verbindung nach Anspruch 1, wobei R¹ eine Methylgruppe darstellt und die Reste R² und R³ Wasserstoffatome darstellen.

5. Pharmazeutische Zusammensetzung, die eine oder mehrere Verbindungen der Formel I oder Salze dieser Verbindungen umfasst, wobei zusätzlich Zusatzstoffe und/oder andere Wirkstoffe enthalten sein können.

6. Pharmazeutische Zusammensetzung nach Anspruch 5, die eine Verbindung der Formel I oder ein Salz dieser Verbindung umfasst, wobei R¹ eine Methylgruppe darstellt und die Reste R² und R³ Wasserstoffatome darstellen.

7. Pharmazeutische Zusammensetzung nach Anspruch 5 oder 6, wobei die Verbindung der Formel I in einer Stoffmenge von mindestens 20 % der Wirkstoffe enthalten ist.

8. Verwendung einer Verbindung der Formel I zur Herstellung einer Infusionslösung, wobei die Reste R¹, R² und R³ wie in Anspruch 1 definiert sind.

9. Verwendung einer Verbindung der Formel I zur Herstellung einer pharmazeutischen Zusammensetzung zur Behandlung von Schmerzen, wobei die Reste R¹, R² und R³ wie in Anspruch 1 definiert sind.

10. Verfahren zur Herstellung von Verbindungen der Formel I: 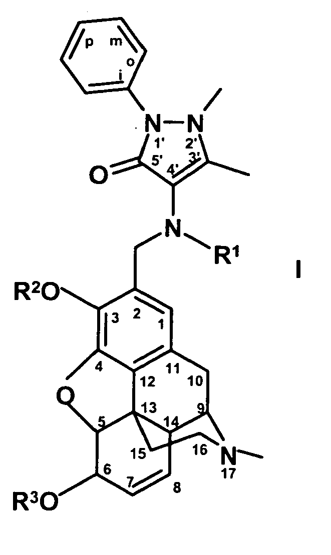 worin eine Verbindung der Formel II, oder ein Salz davon 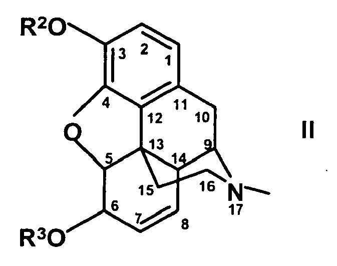 und eine Verbindung der Formel III, oder ein Salz davon 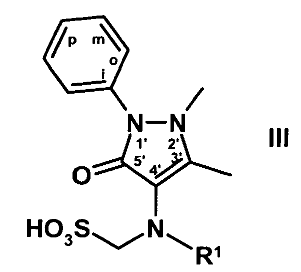 eingesetzt werden, und wobei R¹ einen C₁₋₆-Alkylrest darstellt und die Reste R² und R³ unabhängig voneinander aus der Gruppe ausgewählt sind, die aus Wasserstoffatomen, Methyl- und Acetylgruppen besteht.

11. Verfahren nach Anspruch 10, wobei R¹ eine Methylgruppe darstellt und die Reste R² und R³ Wasserstoffatome darstellen.
